Europäisches Patentamt

European Patent Office

Office européen des brevets

(19)

(11) EP 0 639 371 B1

(12) **FASCICULE DE BREVET EUROPEEN**

(45) Date de publication et mention
de la délivrance du brevet:
**17.06.1998 Bulletin 1998/25**

(51) Int. Cl.⁶: **A61K 7/50**

(21) Numéro de dépôt: **94401852.2**

(22) Date de dépôt: **12.08.1994**

(54) **Composition cosmétique à base de microdispersion de cire comprenant un composé organofluoré lipophile**

Kosmetische Zusammensetzung auf der Basis einer Wax-Mikrodispersion, enthaltend einem lipophilen Organofluorverbindung

Cosmetic composition based on a wax microdispersion comprising an organofluorinated lipophilic compound

(84) Etats contractants désignés:
**AT BE CH DE ES FR GB IT LI NL SE**

(30) Priorité: **20.08.1993 FR 9310160**

(43) Date de publication de la demande:
**22.02.1995 Bulletin 1995/08**

(73) Titulaire: **L'OREAL**
**75008 Paris (FR)**

(72) Inventeurs:
• **Mellul, Myriam**
**F-94240 L'Hay Les Roses (FR)**
• **Piot, Bertrand**
**F-92250 La Garenne-Colombes (FR)**

(74) Mandataire:
**Tonnellier, Jean-Claude**
**Nony & Associés,**
**29, rue Cambacérès**
**75008 Paris (FR)**

(56) Documents cités:
**EP-A- 0 394 078        EP-A- 0 557 196**
**WO-A-93/11103         DE-A- 2 052 579**
**US-A- 3 952 066**

• **PATENT ABSTRACTS OF JAPAN vol. 12, no. 84 (C-482) 17 Mars 1988 & JP-A-62 223 105 (KANEBO LTD) 1 Octobre 1987**
• **Dictionary of Colloid Surface Science, page 102**
• **Microemulsions, Theory and Practice, pages 126-128 et 142-144**

## Description

La présente invention se rapporte à une composition comprenant une dispersion aqueuse de particules de cire et au moins un composé organofluoré lipophile, la dispersion étant une microdispersion aqueuse d'au moins une cire. Une telle composition est utilisable notamment comme composition cosmétique.

On sait que les cires, dont l'utilisation en cosmétologie est très ancienne, sont des substances naturelles (animales ou végétales) ou synthétiques, solides à la température ordinaire (21°C), qui sont insolubles dans l'eau, solubles dans les huiles, et qui sont capables de former des films hydrofuges. Sur la définition des cires et leurs utilisations en cosmétologie, on peut citer par exemple P.D. Dorgan, Drug and Cosmetic Industry, Décembre 1983, pages 30-33, et Handbook of Cosmetic Science, H.W. Hibbot ed., Pergamon Press, Oxford (1963) page 60. Dans les préparations capillaires, l'utilisation la plus classique est celle de préparations semi-solides appelées pommades pour cheveux ou brillantines solides. Dans de telles compositions, les cires sont utilisées en mélange notamment avec des proportions importantes d'huiles diverses ; voir par exemple E.W. Flick, "Cosmetic and Toiletry Formulations" Ed. Noyes Publication-New Jersey-USA (1984) pages 271-288.

On sait par ailleurs qu'il est possible d'obtenir avec certaines huiles des microémulsions et avec certaines cires des microdispersions stables et diluables à l'eau indéfiniment, sans agrégation ni sédimentation des particules en suspension. Les microdispersions de cire sont obtenues par fusion de la cire en présence d'un tensio-actif, et éventuellement d'une partie de l'eau, puis addition progressive d'eau chaude avec agitation. On observe la formation intermédiaire d'une émulsion du type eau-dans-l'huile, suivie d'une inversion de phase avec obtention finale d'une émulsion du type huile-dans-l'eau. Au refroidissement, on obtient une microdispersion stable de particules colloïdales solides de cire; voir par exemple "Microemulsions Theory and Practice", L.M. Prince Ed., Academic Press (1977) pages 21-32.

Les microdispersions de cires peuvent être utilisées par exemple comme agents d'entretien (produits auto-lustrants) des articles en cuir, des revêtements de sols (notamment en plastique), ou des meubles, ou encore comme agents de conditionnement des textiles.

On a également décrit, dans les demandes de brevet EP-A-394.078, EP-A-446.094 et EP-A-477.053 des microdispersions de cires destinées à être appliquées sur les cheveux.

Ces microdispersions de cires conduisent, après application, à des films cireux présentant une certaine rigidité. Il est souhaitable que ces propriétés de rigidité soient modifiées pour conférer au film une meilleure plasticité.

On a découvert que l'addition de composés organofluorés hydrocarbonés lipophiles dans une microdispersion de cire permettait de plastifier les microparticules de cires.

On a ainsi découvert qu'après addition de certains composés organofluorés lipophiles, des compositions à base de microdispersions aqueuses de cire présentent de bonnes propriétés filmogènes et plastiques qui s'avèrent intéressantes notamment pour l'application sur les cils, sur les cheveux, ainsi que sur les fibres textiles, et pour l'application sur les cuirs, revêtements de sol ou meubles, pour lesquels l'effet lustrant obtenu est amélioré et est plus durable.

Alors que les composés organofluorés lipophiles perfluorés ne sont pas compatibles avec les microdispersions de cires (ils ne donnent pas avec les cires de microparticules solides homogènes), on a découvert que, de façon surprenante, les composés organofluore's lipophiles partiellement fluorés sont compatibles avec les microémulsions de cires dont ils améliorent les propriétés comme indiqué ci-dessus.

Le document EP-A-0557196 décrit une composition cosmétique pour le maquillage des yeux, comprenant une microdispersion de cire. Les exemples E et F de ce document décrivent deux compositions particulières contenant respectivement une huile fluorée, et une cire fluorée, dont le rôle n'est pas indiqué.

Le document Patent Abstracts of Japan vol.12, n°84 (C-482) décrit une émulsion triple perfluorodécaline/phase huileuse/phase aqueuse utilisable comme cosmétique. La phase huileuse, qui contient un tensioactif fluoré, peut aussi contenir une cire.

La présente invention, dans un de ses aspects, a donc pour objet une composition, et notamment une composition cosmétique ou un support de composition cosmétique, comprenant une microdispersion aqueuse de particules contenant au moins une cire et au moins un composé lipophile organofluoré.

Les compositions de l'invention sont exemptes de composés organofluorés perfluorés.

L'invention a notamment pour objet une composition comprenant une microdispersion aqueuse de particules contenant au moins une cire, lesdites particules étant solides jusqu'à une température au moins égale à 30°C, caractérisée par le fait que lesdites particules contiennent en outre un composé organique lipophile hydrofluorocarboné tel que le rapport du nombre d'atomes de fluor au nombre total d'atomes de fluor et d'hydrogène liés aux atomes de carbone, dans la molécule dudit composé est au moins égal à 10 % et n'est pas supérieur à 90 %,

à l'exception d'une composition contenant de la cire de carnauba, un composé organofluoré, un tensioactif non-ionique et du parahydroxybenzoate de méthyle dans les proportions pondérales relatives p1:p2:p3:p4, respectivement, ledit tersioactif non-ionique étant un monostéarate de glycérol polyoxyéthyléné à 30 moles d'oxyde d'éthylène et ledit composé organofluoré étant choisi parmi (a) le 1-(2'-F-hexyléthylthio)-3-(2''-éthylhexyloxy) propane-2-ol et (b) le 1-(2'-F-octyléthylthio)2-hexanol,

étant entendu que :

dans le cas (a) lesdites proportions pondérales relatives p1:p2:p3:p4 sont : 22,5:7,5:7,5:0,2, respectivement, et que dans le cas (b) lesdites proportions pondérales relatives sont 18:12:7,5:0,2, respectivement.

Les particules de la microdispersion de cire ont des dimensions moyennes inférieures à 1 μm, de préférence inférieures à 0,5 μm.

Ces particules sont constituées essentiellement de la cire ou du mélange de cires et du composé organofluoré hydrocarboné lipophile. Les particules de la microdispersion peuvent en outre contenir en proportion minoritaire des additifs gras huileux ou pâteux, un ou plusieurs tensioactifs et un ou plusieurs ingrédients actifs liposolubles usuels, comme cela sera précisé ci-après.

La composition contient généralement de 1 à 40 % en poids de cires, en particulier 5 à 30 %, et une quantité suffisante d'au moins un émulsionnant. La quantité d'émulsionnant est une quantité suffisante pour permettre d'obtenir une microdispersion de cires telle que définie ci-dessus. Cette quantité suffisante peut être déterminée dans chaque cas par des expériences de routine.

Le point de fusion de la cire ou du mélange de cires est de préférence compris entre 50°C et 100°C.

La cire ou les cires constituant le mélange cireux sont choisies notamment, parmi la cire de Carnauba, la cire de Candelilla, et la cire d'Alfa, et leurs mélanges.

Outre les cires citées ci-dessus, le mélange de cires peut également contenir une ou plusieurs des cires ou famille de cires suivantes :

- la cire de paraffine ;
- l'ozokérite ;
- les cires végétales comme la cire d'olivier, la cire de riz, la cire de jojoba hydrogénée ou les cires absolues de fleurs telles que la cire essentielle de fleur de cassis vendue par la Société Bertin (France) ;
- les cires animales comme les cires d'abeilles, ou les cires d'abeilles modifiées (cerabellina) ;
- d'autres cires ou matières premières cireuses : les cires marines telles que celle vendue par la Société Sophim sous la référence M82, les céramides naturels ou de synthèse, ou les cires de polyéthylène.

Les cires végétales de Carnauba (extraite de *Copernica cerifera*), de Candelilla (extraite de *Euphorbies cerifera* et de *Pedilantus pavonis*) et d'Alfa (extraite de *Stipa tenacissima*), sont des produits commerciaux. Les céramides sont les principaux lipides constitutifs des espaces intercornéocytaires du stratum corneum. Ils sont décrits en particulier par Downing dans Science, 1982, Vol.18,p.1261-2. Des analogues synthétiques sont également connus, tels que les céramides HO3 vendus par la Société Cosmind. Dans le mélange de cires, la cire de Carnauba et/ou de Candelilla et/ou d'Alfa représente de préférence au moins 20 %, et en particulier au moins 50 % en poids par rapport au poids total du mélange de cires.

Dans les microparticules de la composition selon l'invention, la cire et le composé organofluoré lipophile forment un mélange homogène, ledit composé organofluoré étant soluble dans la cire. Dans la composition de l'invention, le composé organofluoré lipophile est généralement présent à raison de 5 à 50%, et en particulier de 10 à 30%, en poids par rapport au poids total de cire.

On considère ici qu'un composé est lipophile s'il est hydrophobe, c'est-à-dire insoluble dans l'eau et miscible avec les lipides usuels et notamment avec les cires telles que définies ci-dessus. En particulier, on peut choisir le composé organofluoré lipophile parmi ceux qui sont miscibles avec la cire de carnauba à un taux d'au moins 10 % en poids.

La cire ou le mélange de cires peut contenir, outre les cires mentionnées ci-dessus, au moins une autre cire et/ou au moins une huile, étant entendu que le mélange de cire(s), de composé organofluoré, et éventuellement d'huile doit être solide jusqu'à une température de 50°C environ et doit fondre à une température inférieure à 100°C.

Le mélange de cires peut donc être associé à un ou plusieurs additifs gras (huileux ou pâteux). On citera de manière non restrictive :

- les huiles végétales comme l'huile de tournesol, l'huile de jojoba, etc. ;
- les huiles minérales comme l'huile de paraffine,
- les huiles de silicones fluides de viscosité comprise notamment entre 0,65 et 100.000 centistokes (soit entre 0,65.10$^{-6}$ et 0,1 m$^2$.s$^{-1}$), de préférence entre 5 et 5 000 centistokes (soit entre 5.10$^{-6}$ et 5.10$^{-3}$ m$^2$.s$^{-1}$),
- la vaseline,
- la lanoline.

Le mélange d'huile(s) et/ou d'additifs gras pâteux peut représenter jusqu'à 30% (de préférence au plus 10%) du poids de cire(s).

Il est possible d'introduire en outre dans la phase cireuse microparticulaire des ingrédients actifs liposolubles.

Lorsqu'ils sont présents, le ou les ingrédients liposolubles représentent au maximum 30%, de préférence au maximum 10%, du poids des microparticules.

Comme ingrédient(s) liposoluble(s), on citera par exemple, dans le cas des compositions cosmétiques :

- des filtres U.V.
- des vitamines liposolubles,
- des anti-inflammatoires tels que l'acide $\beta$-glycyrrhétinique
- des extraits végétaux liposolubles.

Les composés organofluorés utilisables dans les compositions de l'invention ont une structure chimique comportant principalement un squelette hydrocarboné dans lequel une partie des atomes d'hydrogène ont été substitués par des atomes de fluor. Ledit squelette hydrocarboné peut comporter en outre un ou plusieurs hétéroatomes et un ou plusieurs groupements fonctionnels.

Pour une molécule de composé organofluoré hydrocarboné, on définit un taux de substitution des atomes d'hydrogène par des atomes de fluor, sous la forme du rapport : nombre d'atomes de fluor/(nombre d'atomes de fluor + nombre d'atomes d'hydrogène) où seuls les atomes d'hydrogène liés aux atomes de carbone du squelette sont pris en compte dans la mesure considérée.

Pour les composés organofluorés utilisés selon l'invention, ce taux de substitution ne dépasse pas 90 % et est généralement compris entre 10 et 80 %. De façon préférée, ce taux est supérieur à 30 % et inférieur à 60 %.

Les composés organofluorés utilisés dans les compositions de l'invention sont notamment des composés répondant à la formule suivante :

$$(R_F)_x(A)_y(R_H)_z$$

dans laquelle :

$R_F$ représente un groupement organique fluoré ayant au moins 4 atomes de carbone,
$R_H$ représente un groupement hydrocarboné non fluoré, ledit groupement hydrocarboné étant éventuellement substitué et/ou comportant éventuellement un ou plusieurs hétéroatomes ou groupements hétéroatomiques,
A représente un groupement de liaison divalent, trivalent ou tétravalent,
x représente le nombre 1, 2 ou 3,
y représente le nombre 0 ou 1
z représente le nombre 0, 1, 2 ou 3,
étant entendu :
que, lorsque y est différent de zéro, la somme (x + z) est égale à la valence du groupement de liaison A, et alors chaque groupement $R_F$ et $R_H$ est lié à A par une liaison covalente,
que, lorsque y = 0, alors x = z = 1 et le groupement $R_F$ et le groupement $R_H$ sont alors liés l'un à l'autre par une liaison covalente, et
que lorsque z est égal à 0, y est différent de 0.

Dans la définition ci-dessus, les hétéroatomes désignent notamment -O-ou -S- ; les groupements hétéroatomiques sont par exemple des groupements -SO-, -SO$_2$-ou -N(R')-, R' étant notamment un hydrogène, un alkyle ou un aralkyle.

Parmi les composés organofluorés, on peut citer notamment ceux qui présentent une ou plusieurs des caractéristiques suivantes :

a) $R_F$ est un groupement fluorocarboné aliphatique, éventuellement insaturé, ou aromatique, pouvant comporter un ou plusieurs hétéroatomes ou groupements hétéroatomiques et/ou pouvant être substitué par un ou plusieurs atomes d'halogène autre que le fluor, étant entendu que le rapport, en %, du nombre d'atomes de fluor au nombre total d'atomes d'halogène présents dans $R_F$ n'est pas inférieur à 75 % ;
b) $R_F$ est un groupement perfluoroalkyle ayant de 4 à 22 atomes de carbone ;
c) le groupement A est choisi parmi un carbone (tétravalent), un groupement $\equiv$CH (trivalent), un azote (trivalent), un groupement -CO-N= (trivalent), un groupement - SO$_2$N= (trivalent), un groupement PO-(O)$_3$- (trivalent), ou un groupement divalent choisi parmi - CH$_2$-, -O-, -S-, -SO-, -SO$_2$- et -CO-NH-. Il convient de noter que l'indication des valences de A, telle que faite ci-dessus, ne préjuge pas de la structure formée par les liaisons chimiques avec A dans le composé de formule I : par exemple, le groupement $\equiv$CH peut être un groupement lié par 3 liaisons cova-

4

lentes simples ou un groupement lié par une liaison simple et une double liaison (c'est-à-dire du type -CH=);

d) $R_H$ représente un groupement aliphatique, notamment alkyle, saturé ou insaturé en $C_1$-$C_{22}$, aryle en $C_6$-$C_{10}$ ou aralkyle en $C_7$-$C_{15}$, ledit groupement étant éventuellement substitué et/ou interrompu par des hétéroatomes ou groupements hétéroatomiques tels que ceux définis précédemment;

e) les groupements $R_F$ et/ou $R_H$ peuvent être substitués de façon à porter des fonctions telles que : alcool, thiol, fonction carbonylée, acide carboxylique, ester, amine, ènamine, amide, sulfonamide, sulfoxyde, phosphate, etc...;

f) le composé organofluoré lipophile répond à la formule (II):

$$R_1\text{-}(CH_2)_n\text{-}X\text{-}[C_3H_5(OH)]\text{-}(Y)_p\text{-}R_2 \qquad (II)$$

dans laquelle:

$R_1$ représente un radical alkyle perfluoré ayant de 4 à 20 atomes de carbone,
$R_2$ représente un groupement alkyle en $C_1$-$C_{22}$, aryle en $C_6$-$C_{10}$ ou aralkyle en $C_7$-$C_{15}$,
X et Y représentent indépendamment un groupement choisi parmi -O-, -S-, -SO-, -SO$_2$-,
n représente un nombre de 0 à 4,
p représente le nombre 0 ou 1,
et $C_3H_5(OH)$ représente l'un des groupements -CH$_2$CH(OH)CH$_2$-, -CH(CH$_2$OH)-CH$_2$- et -CH$_2$-CH(CH$_2$OH)-,
étant entendu que X et Y ne peuvent pas représenter simultanément -SO-ou -SO$_2$-. Le groupement $R_2$ peut représenter les caractéristiques données pour $R_H$ ci-dessus.

De tels composés sont décrits notamment dans les demandes de brevet WO-93/11103 et EP-166.696;

g) le composé organofluoré répond à la formule (III):

$$R_F\text{-}CH\text{=}CH\text{-}CH_2OCH_2\text{-}[C_2H_4\text{-}OW] \qquad (III)$$

dans laquelle:

$C_2H_4$-OW désigne -CHOH-CH$_2$W ou -CHW-CH$_2$OH,
W représente -OR, -SR, -COOR, -OC$_6$H$_5$ ou -O-C$_6$H$_4$-R",
R représente un groupement alkyle ayant de 1 à 18 atomes de carbone,
R" représente -CH$_3$ ou -OH en position ortho ou para, et
$R_F$ représente un radical alkyle perfluoré ayant de 4 à 20 atomes de carbone.

Ces composés sont décrits notamment dans le brevet US-3952066

h) le composé organofluoré lipophile répond à la formule (IV):

$$R_F\text{-}CH_2\text{-}CH_2\text{-}X\text{-}CH_2\text{-}CH(Y')\text{-}Z \qquad (IV)$$

dans laquelle:

$R_F$ est un groupement alkyle perfluoré ayant de 4 à 20 atomes de carbone,
X représente -O-, -S-, -SO- ou -SO$_2$-,
soit Y' représente OH et Z représente alors -C$_6$H$_5$, -CH$_3$, -CH$_2$OH, -CH$_2$O-COCH$_3$,
soit Y' représente -CH$_2$OH et Z représente alors -O-COCH$_3$.

Ces composés sont décrits notamment dans le document DE-2052079;

i) le composé organofluoré lipophile répond à la formule (V):

$$C_rF_{2r+1}\text{-}(CH_2)_p\text{-}O\text{-}CO\text{-}(CH_2)_7\text{-}CH\text{=}CH\text{-}(CH_2)_7\text{-}CH_3 \qquad (V)$$

dans laquelle r est un nombre entier égal à 6 ou 8 et p est le nombre 1 ou 2.

Parmi ces composés, on citera ceux commercialisés sous la dénomination Nofable FO par la Société Nippon Oil & Co.;

j) le composé organofluoré lipophile répond à la formule:

$$C_rF_{2r+1}\text{-}(CH_2)_v\text{-}CH_3$$

dans laquelle r est un nombre entier égal à 6 ou 8 et v est un nombre entier pouvant varier de 12 à 16 ; ces composés sont décrits par M. Napoli, IX European Symposium of Fluorine Chemistry, Leicester, Septembre 1989;

k) le composé organofluoré lipophile peut aussi être choisi parmi ceux décrits dans JP-63-002.916, qui possèdent des groupements perfluorocarbonés et des groupements hydrocarbonés, le nombre total d'atomes de carbone étant compris entre 10 et 30, et le nombre d'atomes de carbone des groupements hydrocarbonés étant égal ou supérieur à 2 fois le nombre d'atomes de carbone des groupements perfluorcarbonés.

L'utilisation de tensioactifs comme émulsionnants dans la préparation de microdispersions de cires est connue. La réalisation de la microdispersion peut être effectuée à l'aide de tensioactifs anioniques, cationiques et/ou non-ioniques, de façon connue.

La quantité de tensioactif présent dans la composition est une quantité au moins suffisante pour permettre la formation d'une microdispersion stable du mélange de cire et de composé organofluoré. Cette quantité peut être déterminée dans chaque cas par des expériences de routine.

Le pourcentage pondéral de tensio-actif(s) dans la composition finale est compris généralement entre 0,01 et 25% environ et en particulier peut varier de 0,1 à 10%.

Le rapport pondéral cire(s)/émulsionnant(s) peut varier par exemple dans la gamme de 1 à 30 et notamment de 2 à 10.

Les tensioactifs anioniques utilisés sont notamment des sels d'acides gras (par exemple sels alcalins ou sels organiques tels que les sels d'amines) lesdits acides gras, éventuellement insaturés, ayant par exemple de 12 à 18 atomes de carbone, des sels alcalins ou sels de bases organiques des acides alkyl-sulfuriques et alkylsulfoniques ayant 12 à 18 atomes de carbone ou d'acides alkyl-arylsulfoniques dont la chaîne alkyle contient de 6 à 18 atomes de carbone. On peut citer également les éthers-sulfates, en particulier les produits de sulfatation des alcools gras et alkylphénols polyalcoxylés dans lesquels la chaîne aliphatique comporte de 6 à 20 atomes de carbone et la chaîne polyalcoxylée de 1 à 30 motifs oxyalkylène, en particulier oxyéthylène, oxypropylène ou oxybutylène.

Tous ces tensioactifs anioniques sont bien connus et plusieurs d'entre eux sont des produits commerciaux.

Les tensio-actifs non ioniques sont par exemple des tensio-actifs polyalcoxylés et/ou polyglycérolés. Ce sont notamment les acides gras ou les amides d'acide gras polyalcoxylés et/ou polyglycérolés ; les alcools gras ou les alkylphénols polyalcoxylés et/ou polyglycérolés ; les esters d'acides gras et de polyols polyalcoxylés et/ou polyglycérolés ; les alcanediols ou alcènediols-1,2 ou -1,3 polyalcoxylés et/ou polyglycérolés ; et les alkyléthers d'alcanediols ou alcènediols-1,2 ou -1,3 polyalcoxylés et/ou polyglycérolés ; par exemple, les acides ou alcools gras, éventuellement insaturés, ont 12 à 24 atomes de carbone, la chaîne alkyle des alkylphénols a 6 à 16 atomes de carbone, les alcanediols ou alcènediols ont de 9 à 24 atomes de carbone, l'alkyle des alkyléthers a de 4 à 20 atomes de carbone, et le nombre de motifs oxyalkylène ou de motifs $(CH_2CHOHCH_2O)$ peut aller de 2 à 40.

Les dérivés non ioniques polyalcoxylés sont notamment des dérivés polyoxyéthylénés, éventuellement polyoxypropylénés.

Les acides gras polyalcoxylés sont des produits commerciaux, notamment les produits vendus sous la marque Myrj par la Société Atlas.

Les esters d'acides gras et de polyols polyoxyéthylénés pour lesquels le polyol est le sorbitol sont des produits connus (Polysorbate et produits commercialisés sous la marque Tween par la Société Atlas). Lorsque le polyol est le glycérol, on peut utiliser les produits commercialisés par le Société Goldschmidt sous la marque Tagat.

Les alcools gras polyoxyéthylénés sont également des produits commerciaux, notamment ceux vendus sous la marque Brij par la Société Atlas.

Les alcools gras polyglycérolés, les alcanediols ou alcènediols polyglycérolés, ou les alkyléthers d'alcanediols ou d'alcènediols polyglycérolés peuvent être préparés par exemple selon les procédés décrits dans les brevets français 1.477.048, 2.025.681, 2.091.516 et 2.465.780 ou selon des procédés analogues.

Les acides gras ou amides d'acides gras polyglycérolés sont notamment décrits dans le brevet français 1.484.723 ou sont encore des produits commerciaux tels que ceux vendus sous la marque Plurol (Gattefosse) ou Drewpol, (Stefan Company), ou Decaglyn (Nikko Chemical).

D'autres tensioactifs non-ioniques utilisables sont par exemple:

- les alkylcarbamates de triglycérol de formule générale:

$$R\text{-}NHCOOCH(CH_2OCH_2CHOHCH_2OH)_2$$

dans laquelle R représente un groupe alkyle saturé ou non de 10 à 20 atomes de carbone. Ces composés sont décrits dans le brevet EP-0.420.761 ;

- les dérivés oxyéthylénés ou propoxylés des alcools de la lanoline, des acides gras de la lanoline, ou de leurs mélanges.

De tels tensioactifs sont commercialisés par la Société Amerchol sous la marque Solulan.

Les tensioactifs cationiques sont notamment les dérivés d'ammonium quaternaire tels que l'Arquad 16-50, l'Arquad 18-50, l'Arquad T-50, l'Arquad 2C-75, l'Ethoquad c/12, et l'Ethoquad o/12, commercialisés par la Société Armak Chemicals.

L'emploi des tensioactifs non ioniques est préféré.

Il est également possible de mélanger les microdispersions de cire et de composé organofluoré avec des mélanges commerciaux de cires auto-émulsionnables contenant une cire et des tensioactifs. On peut utiliser par exemple la cire commercialisée sous la dénomination Cire Auto Lustrante OFR par la Société Tiscco, qui contient des cires de Carnauba et de paraffine, en association avec des agents émulsionnants non ioniques, ou la cire auto-émulsionnable commercialisée sous la dénomination Cerax A.O. 28/B par La Ceresine, qui contient de la cire d'Alfa en association avec un émulsionnant non ionique. Ces mélanges commerciaux permettent de préparer des microdispersions de cires par addition d'eau selon le procédé décrit ci-dessus.

On peut également utiliser des microdispersions de cires prêtes à l'emploi disponibles commercialement comme les produits de la série SL slipaid de la Société Daniel Products Company, ou encore les produits Aquacer de la société Cerachemie.

Les microdispersions de cires sont diluables à l'eau sans nuire à la stabilité de la microdispersion. Elles peuvent donc se présenter sous la forme de compositions concentrées dont on peut ajuster la proportion des ingrédients à une valeur désirée par simple addition d'eau.

La composition selon l'invention peut contenir des polymères filmogènes.

Les polymères filmogènes utilisables sont notamment ceux utilisés habituellement dans les compositions cosmétiques pour les cheveux et les cils. Ce sont par exemple les polymères décrits dans les documents suivants : FR-1.222.944, FR-1.492.597, FR-1.564.110, FR-1.580.545, FR-2.077.141, FR-2.080.759, FR-2.137.684, FR-2.162.025, FR-2.190.406, FR-2.198.719, FR-2.265.781, FR-2.265.782, FR-2.280.361, FR-2.350.834, FR-2.357.241, FR-2.393.573, FR-2.403.076, FR-2.439.798, FR-2.529.214, BE-208.516, GB-839.805, US-2.047.398, US-2.102.113, US-2.723.248, US-3.589.978, US-3.879.376, US-4.031.307, US-4.082.730, US-4.128.631 et US-4.131.576. Les polymères filmogènes peuvent être présents dans la composition à raison de 0 à 25 % en poids, et en particulier de 2 à 15 %.

La composition peut contenir également des pigments colorés ou des charges utilisés de façon usuelle.

De tels pigments colorés ou charges (non colorées) sont présents en quantité suffisante, généralement inférieure à 40 % en poids par rapport au poids total de la composition. On peut utiliser des pigments organiques ou minéraux, y compris des pigments nacrés. On citera par exemple le dioxyde de titane, les oxydes de fer, l'oxyde de chrome ; le noir de carbone et les divers pigments organiques D et C red, orange ou yellow codifiés dans le Color Index ; les pigments nacrés tels que le mica recouvert d'oxyde de titane ; les charges telles que le talc, les micas, l'amidon, les oxydes de zinc et de titane, le carbonate de calcium, les poudres de polymères synthétiques (polyéthylène, polyamide, etc...) et les poudres de silicone.

La composition peut contenir également d'autres additifs tels que des colorants, des filtres solaires, des agents épaississants, des parfums, des conservateurs, etc...

Les compositions selon l'invention peuvent se présenter sous forme de lotion fluide ou de lotion légèrement épaissie ou gélifiée. Elles peuvent être utilisées pour une application capillaire : lotion de mise en plis, lotion de coiffage, ou pour le maquillage des yeux : mascara, eye-liner (ou "ligneur").

Lorsque la composition est utilisée pour le maquillage des yeux, la viscosité de la composition est comprise par exemple entre 2,5 Pa.s et 35 Pa.s et de préférence entre 3,5 et 25 Pa.s. (mesurée au viscosimètre Contraves à 200 t/min après 10 minutes de rotation).

Les compositions selon l'invention sont obtenues par formation à chaud d'une microémulsion. Plus précisément, ces compositions sont obtenues par un procédé principalement caractérisé par le fait que l'on chauffe la cire et l'émulsionnant à une température supérieure à la température de fusion de la cire et non supérieure à 100°C, éventuellement en présence d'une partie de l'eau, jusqu'à fusion complète de la cire, que l'on ajoute progressivement l'eau, ou le restant de l'eau, portée à une température au moins égale à ladite température, en agitant, jusqu'à formation d'une microémulsion de cire dans une phase continue aqueuse, puis que l'on laisse refroidir jusqu'à la température ambiante. On obtient une microdispersion stable de cire.

On opère sous une agitation et en présence d'une quantité de tensio-actif suffisantes pour que les dimensions des microparticules de cire soient inférieures à 1 micromètre et de préférence inférieures à 500 nm.

Les composés organofluorés et les ingrédients liposolubles, par exemple des céramides, sont généralement ajoutés à la cire avant la réalisation de la microdispersion.

Les ingrédients hydrosolubles peuvent être ajoutés dans l'eau utilisée pour réaliser la microdispersion, ou dans la microdispersion de cire finalement obtenue.

De façon analogue, les ingrédients secondaires éventuellement présents dans la composition sont ajoutés selon les cas soit dans les produits de départ, soit dans la composition terminée.

On peut également ajouter à la microdispersion obtenue une microdispersion de cire commerciale, comme indiqué précédemment.

L'invention a également pour objet un procédé de traitement cosmétique, caractérisé par le fait que l'on applique sur les cheveux ou sur les cils une composition cosmétique telle que définie ci-dessus. L'application est effectuée de façon connue en soi.

L'invention a en outre pour objet l'utilisation d'un composé organique lipophile hydrofluorocarboné tel que le rapport du nombre d'atomes de fluor au nombre total d'atomes de fluor et d'hydrogène liés aux atomes de carbone, dans la molécule dudit composé, est au moins égal à 10 % et n'est pas supérieur à 90 %, comme additif destiné à améliorer les propriétés d'une composition cosmétique comprenant une microdispersion aqueuse de cire.

La composition cosmétique et le composé hydrofluorocarboné peuvent être tels que définis précédemment. L'additif fluoré améliore notamment les propriétés cosmétiques et la plasticité des films obtenus par application de ces compositions sur la peau.

Les exemples suivants illustrent l'invention.

**EXEMPLES DE PREPARATION DE MICRODISPERSIONS DE CIRES**

<u>**EXEMPLE A**</u> :

| | |
|---|---|
| - Cire de carnauba | 22,5 g |
| - 1-(2'-F hexyléthylthio)-3-(2''-éthyl hexyloxy) propane 2-ol | 7,5 g |
| - Monostéarate de glycérol oxyéthyléné à 30 moles d'oxyde d'éthylène vendu sous la dénomination "Tagat S" par la Société Goldschmidt | 7,5 g |
| - Conservateurs qs | |
| - Eau qsp | 100,0 g |

On chauffe à 90°C le mélange de cire, de composé fluoré et de tensioactifs en homogénéisant sous agitation modérée.

En continuant d'agiter, on incorpore l'eau préalablement chauffée à 90°C.

La microémulsion obtenue est ramenée à température ambiante. On ajoute les conservateurs lorsque la température est voisine de 30°C.

Diamètre moyen des particules de cire : 250 nm.

**EXEMPLE B** :

| | |
|---|---|
| - Cire de carnauba | 10,0 g |
| - Alcool de lanoline et mélange d'alcools gras polyoxyéthylénés à 25 moles d'oxyde d'éthylène vendu sous la dénomination de "Solulan 25" par la Société Amerchol | 7,5 g |
| - Parahydroxybenzoate de méthyle | 0,3 g |
| - Composé fluoré vendu sous la dénomination "Nofable FO"* par la Société Nippon Oil | 2,5 g |
| - Eau déminéralisée qsp | 100 g |

\* La formule de ce composé fluoré est :

$$C_8F_{17}\text{-}(CH_2)_2\text{-}O\text{-}CO\text{-}(CH_2)_7\text{-}CH=CH\text{-}(CH_2)_7\text{-}CH_3$$

La microdispersion est obtenue en procédant comme décrit dans l'exemple A.
Diamètre moyen des particules de cire : 111 nm.

**EXEMPLE C** :

| | |
|---|---|
| - Cire de carnauba | 10,0 g |
| - Alcool cétylique polyoxyéthyléné à 20 moles d'oxyde d'éthylène vendu sous la dénomination "Brij 58" par la Société ICI | 2,34 g |
| - Composé fluoré "Nofable FO" | 2,5 g |
| - Parahydroxybenzoate de méthyle | 0,3 g |
| - Eau déminéralisée qsp | 100,0 g |

La microdispersion est obtenue en procédant comme décrit dans l'exemple A.
Diamètre moyen des particules : 118 nm.

**EXEMPLE D** :

| | |
|---|---|
| - Cire de carnauba | 10,0 g |
| - Bromure de cétyltriméthylammonium | 3,79 g |
| - Composé fluoré "Nofable FO" | 2,50 g |
| - Parahydroxybenzoate de méthyle | 0,3 g |
| - Eau qsp | 100,0 g |

La microdispersion est obtenue comme décrit dans l'exemple A.
Diamètre moyen des particules : 96 nm.

**EXEMPLE E** :

| - Cire de carnauba | 18,0 g |
|---|---|
| - "Tagat S" | 7,5 g |
| - Parahydroxybenzoate de méthyle | 0,2 g |
| - 1-(2'-F-hexyléthylthio)-2-décanol | 12,0 g |
| - Eau qsp | 100,0 g |

La microdispersion est préparée selon le mode opératoire de l'exemple A.
Diamètre moyen des particules : 250 nm.

**EXEMPLE F (comparatif)** :

Dans cet exemple, on utilise comme agent organofluoré, un composé perfluoré.

| - Cire de carnauba | 10,0 g |
|---|---|
| - "Solulan 25" | 7,5 g |
| - Parahydroxybenzoate de méthyle | 0,3 g |
| - Pefluoropolyméthylisopropyléther vendu sous la dénomination "HC 25" par la Société Ausimont-Monsanto | 2,5 g |
| - Eau déminéralisée qsp | 100 g |

La microdispersion, qui est préparée selon le procédé décrit à l'exemple A, n'est pas stable, ce qui se traduit notamment par une séparation des phases.

**EXEMPLE G (comparatif)** :

On prépare une microémulsion, sans agent organofluoré, ayant la composition suivante :

| - Cire de carnauba | 30 g |
|---|---|
| - "Tagat S" | 7,5 g |
| - Conservateur qs | |
| - Eau qsp | 100,0 g |

La microdispersion est obtenue selon le procédé décrit à l'exemple A.
Diamètre des particules : 160 nm.

**EXEMPLE H** :

| - Cire de carnauba | 21 g |
|---|---|
| - Cire de paraffine | 3 g |

(suite)

| | |
|---|---|
| - 1-(2'-F-hexyléthylthio)-3-(2"-éthyl hexyloxy) propane 2-ol | 6 g |
| - "Tagat S" | 7,5 g |
| - Conservateurs qs | |
| - Eau qsp | 100,0 g |

La microdispersion est préparée selon le mode opératoire de l'exemple A.

Diamètre moyen des particules : 300 nm.

**EXEMPLE I** :

| | |
|---|---|
| - Cire de carnauba | 19 g |
| - Cire d'abeille modifiée vendue sous la dénomination "Cerabellina" par la Société Jan Dekker | 3 g |
| - 1-(2'-F-hexyléthylthio)-3-(2"-éthyl hexyloxy) propane 2-ol | 8 g |
| - "Tagat S" | 7,5 g |
| - Eau qsp | 100,0 g |

La microdispersion est préparée selon le mode opératoire de l'exemple A.
Diamètre moyen des particules : 250 nm.

**EXEMPLES DE COMPOSITIONS DE MAQUILLAGE POUR LES YEUX**

Mode opératoire des exemples 1 à 4 :

On opère par dilution de la microdispersion de cire de départ en 2 étapes.
A température ambiante, le ou les polymères présents dans la formulation sont incorporés dans la microdispersion de cire sous agitation en ajoutant éventuellement de l'eau, si nécessaire, pour obtenir une préparation homogène. Ensuite, on disperse les pigments. La formulation peut être broyée.

**EXEMPLE 1** :

On prépare la composition suivante :

| | |
|---|---|
| - Microdispersion de cire selon l'exemple A | 89,5 g |
| - Oxyde de fer noir | 5 g |
| - Hydroxyéthylcellulose vendue sous la dénomination de "Cellosize QP 4400M" par la Société Amerchol | 1 g |
| - Gomme arabique | 1,5 g |
| - Panthénol | 1,0 g |
| - NaOH qs pH 7 | |
| - Eau | 2,0 g |

On applique cette composition comme mascara, sur les cils.
A l'application, on constate que les cils sont brillants, possèdent un bon recourbement et une bonne souplesse.

**EXEMPLE 2** :

| | |
|---|---|
| - Microdispersion de cire selon l'exemple E | 86 g |
| - Oxyde de fer noir | 6 g |
| - Glycérine | 3 g |
| - Polyvinylpyrrolidone vendue sous la dénomination de "Luviskol K90" par la Société BASF | 4 g |
| - Polyméthacrylate de sodium | 1 g |
| - NaOH qs pH 7 | |

On applique cette composition de mascara, sur les cils.
A l'application, on constate que les cils sont brillants, possèdent un bon recourbement et une bonne souplesse.

**EXEMPLE 3 ET 4 (comparatifs)** :

On prépare des mascaras de compositions suivantes (les teneurs sont exprimées en grammes) :

| | Ex.3 | Ex.4 |
|---|---|---|
| - Microdispersion de cire selon l'exemple A | 88,5 | |
| - Microdispersion de cire selon l'exemple G | | 88,5 |
| - Oxyde de fer noir | 5,0 | 5,0 |
| - Gomme arabique | 3,0 | 3,0 |
| - "Cellosize QP 400M" | 0,5 | 0,5 |
| - Polyvinylpyrrolidone ("Luviskol K90- BASF) | 1,0 | 1,0 |
| - Polyquaternium-10 (Nom : CTFA) vendu sous la dénomination de "Leogard GP" par la Société Akzo | 2 | 2 |
| - NaOH qs pH7 | | |

On demande à un jury de 10 utilisatrices d'utiliser les mascaras des exemples 3 et 4, dont elles ignorent la composition, et de donner leur opinion sur les qualités ou défauts de ces mascaras. Le mascara de l'exemple 4 est jugé par l'ensemble des utilisatrices comme rigidifiant trop fortement les cils, alors que le mascara de l'exemple 3 est jugé comme rigidifiant normalement les cils.

**EXEMPLE 5** : *Mousse de coiffage*

On prépare une mousse de coiffage aérosol ayant la composition suivante :

| | |
|---|---|
| - Microdispersion de cire de l'exemple A | 22,22 g |
| - Hydroxyéthylcellulose réticulée à l'épichlorhydrine, quaternisée par la triméthylamine, commercialisée sous le nom "J.R. 400" par la Société Union Carbide | 0,5 g |
| - Diazolidinyl urée vendue sous la dénomination "Germall II" par la Société Sutton Labs | 0,1 g |
| - Eau qsp | 100 g |

(suite)

| - NaOH qs pH 5,5 | |
|---|---|

On introduit 90 g de la composition obtenue dans un boitier aérosol sans tube plongeur. On procède à la fixation de la valve et à la fermeture hermétique du récipient, puis on introduit 10 g d'un mélange propulseur butane/isobutane/propane. La pression dans le récipient est 3,2 bars. On rappelle qu'un bar correspond à une pression de $10^5$ Pa.

La mousse de coiffage obtenue est appliquée sur cheveux mouillés. Après séchage, les cheveux présentent du corps et on constate une bonne tenue de la coiffure.

**EXEMPLE 6** :

On prépare un mascara de composition suivante :

| - Microdispersion de cire selon l'exemple B | 93,0 g |
|---|---|
| - Noir de charbon | 5,0 g |
| - Hydroxypropyl chitosane | 0,2 g |
| - Hydroxyéthylcellulose vendue sous la dénomination de "Cellosize QP 4400M" par la Société Amerchol | 1,3 g |
| - α-bisabolol | 0,5 g |

Appliquée sur les cils, cette composition leur confère une brillance, un recourbement et une souplesse satisfaisants.

**Revendications**

1.  Composition cosmétique ou support de composition cosmétique comprenant une microdispersion aqueuse de particules contenant au moins une cire, lesdites particules étant solides jusqu'à une température au moins égale à 30°C, caractérisée par le fait que lesdites particules contiennent en outre un composé organique lipophile hydrofluorocarboné tel que le rapport du nombre d'atomes de fluor au nombre total d'atomes de fluor et d'hydrogène liés aux atomes de carbone, dans la molécule dudit composé est au moins égal à 10 % et n'est pas supérieur à 90 %, la cire et ledit composé organique lipophile formant, dans lesdites particules, un mélange homogène,
    à l'exception d'une composition contenant de la cire de carnauba, un composé organofluoré, un tensioactif non-ionique et du parahydroxybenzoate de méthyle dans les proportions pondérales relatives p1:p2:p3:p4, respectivement, ledit tensioactif non-ionique étant un monostéarate de glycérol polyoxyéthyléné à 30 moles d'oxyde d'éthylène et ledit composé organofluoré étant choisi parmi (a) le 1-(2'-F-hexyléthylthio)-3-(2''-éthylhexyloxy) propane-2-ol et (b) le 1-(2'-F-octyléthylthio)2-hexanol,
    étant entendu que :

    dans le cas (a) lesdites proportions pondérales relatives p1:p2:p3:p4 sont :
    22,5:7,5:7,5:0,2, respectivement,
    et que dans le cas (b) lesdites proportions pondérales relatives sont 18:12:7,5:0,2,
    respectivement.

2.  Composition selon la revendication 1, caractérisée par le fait que ledit rapport est compris dans la gamme de 10 à 80 %, et en particulier de 30 à 60 %.

3.  Composition selon l'une quelconque des revendications précédentes, caractérisée par le fait qu'elle contient de 5 à 50 %, et en particulier de 10 à 30 %, dudit composé organique lipophile hydrofluorocarboné, en poids par rapport au poids total de cire présent dans la composition.

4.  Composition selon l'une quelconque des revendications précédentes, caractérisée par le fait que ledit composé organique lipophile hydrofluorocarboné est miscible avec la cire de carnauba à un taux d'au moins 10 % en poids.

**5.** Composition selon l'une quelconque des revendications précédentes, caractérisée par le fait qu'elle contient de 1 à 40 % en poids, et en particulier de 5 à 30 % en poids de cire.

**6.** Composition selon l'une quelconque des revendications précédentes, caractérisée par le fait que ledit composé organique lipophile hydrofluorocarboné répond à la formule :

$$(R_F)_x(A)_y(R_H)_z \qquad (I)$$

dans laquelle :

$R_F$ représente un groupement organique fluoré ayant au moins 4 atomes de carbone,
$R_H$ représente un groupement hydrocarboné non fluoré, ledit groupement hydrocarboné étant éventuellement substitué et/ou comportant éventuellement un ou plusieurs hétéroatomes ou groupements hétéroatomiques,
A représente un groupement de liaison divalent, trivalent ou tétravalent,
x représente le nombre 1, 2 ou 3,
y représente le nombre 0 ou 1
z représente le nombre 0, 1, 2 ou 3,
étant entendu :
que, lorsque y est différent de zéro, la somme (x + z) est égale à la valence du groupement de liaison A, et alors chaque groupement $R_F$ et $R_H$ est lié à A par une liaison covalente,
que, lorsque y = 0, alors x = z = 1 et le groupement $R_F$ et le groupement $R_H$ sont alors liés l'un à l'autre par une liaison covalente, et
que lorsque z est égal à 0, y est différent de 0.

**7.** Composition selon la revendication précédente, caractérisée par le fait que $R_F$ est un groupement fluorocarboné aliphatique, éventuellement insaturé, ou aromatique, pouvant comporter un ou plusieurs hétéroatomes ou groupements hétéroatomiques et/ou pouvant être substitué par un ou plusieurs atomes d'halogène autre que le fluor, étant entendu que le rapport, en %, du nombre d'atomes de fluor au nombre total d'atomes d'halogène présents dans $R_F$ n'est pas inférieur à 75 %.

**8.** Composition selon la revendication 6, caractérisée par le fait que $R_F$ représente un groupement perfluoralkyle ayant de 4 à 22 atomes de carbone.

**9.** Composition selon l'une quelconque des revendications 6 à 8, caractérisée par le fait que le groupement de liaison A est choisi parmi un carbone (tétravalent), un groupement $\equiv$CH (trivalent), un azote (trivalent), un groupement -CO-N= (trivalent), un groupement -SO$_2$N= (trivalent), un groupement PO-(O)$_3$- (trivalent), ou un groupement divalent choisi parmi -CH$_2$-, -O-, -S-, -SO-, -SO$_2$- et -CO-NH-.

**10.** Composition selon l'une quelconque des revendications 6 à 9, caractérisée par le fait que $R_H$ représente un groupement alkyle, éventuellement insaturé, 1-22 C, aryle 6-10 C ou aralkyle 7-15 C.

**11.** Composition selon l'une quelconque des revendications précédentes, caractérisée par le fait que ledit composé organique lipophile hydrofluorocarboné répond à la formule (II) :

$$R_1\text{-}(CH_2)_n\text{-}X\text{-}[C_3H_5(OH)]\text{-}(Y)_p\text{-}R_2 \qquad (II)$$

dans laquelle :

$R_1$ représente un radical alkyle perfluoré ayant de 4 à 20 atomes de carbone,
$R_2$ représente un groupement alkyle en $C_1$-$C_{22}$, aryle en $C_6$-$C_{10}$ ou aralkyle en $C_7$-$C_{15}$,
X et Y représentent indépendamment un groupement choisi parmi -O-, -S-, -SO-, -SO$_2$-,
n représente un nombre de choisi parmi 0, 1, 2, 3 ou 4,
p représente le nombre 0 ou 1,
et $C_3H_5$(OH) représente l'un des groupements -CH$_2$CH(OH)CH$_2$-, -CH(CH$_2$OH)-CH$_2$- et -CH$_2$-CH(CH$_2$OH)-,
étant entendu que X et Y ne peuvent pas représenter simultanément -SO-ou -SO$_2$-.

**12.** Composition selon l'une quelconque des revendications 1 à 9, caractérisée par le fait que ledit composé organique lipophile hydrofluorocarboné répond à la formule (IV) :

$$R_F\text{-}CH_2\text{-}CH_2\text{-}X\text{-}CH_2\text{-}CH(Y')\text{-}Z \qquad\qquad (IV)$$

dans laquelle :

$R_F$ est un groupement alkyle perfluoré ayant de 4 à 20 atomes de carbone,
X représente -O-, -S-, -SO- ou -SO$_2$-,
soit Y' représente OH et Z représente alors -C$_6$H$_5$, -CH$_3$, -CH$_2$OH, -CH$_2$O-COCH$_3$,
soit Y' représente -CH$_2$OH et Z représente alors -O-COCH$_3$.

13. Composition selon l'une quelconque des revendications 1 à 10, caractérisée par le fait que ledit composé organique lipophile hydrofluorocarboné répond à la formule (III) :

$$R_F\text{-}CH=CH\text{-}CH_2OCH_2\text{-}[C_2H_4\text{-}OW] \qquad\qquad (III)$$

dans laquelle :

C$_2$H$_4$-OW désigne -CHOH-CH$_2$W ou -CHW-CH$_2$OH,
W représente -OR, -SR, -COOR, -OC$_6$H$_5$ ou -O-C$_6$H$_4$-R",
R représente un groupement alkyle ayant 1 à 18 C,
R" représente -CH$_3$ ou -OH en position ortho ou para, et
R$_F$ représente un radical alkyle perfluoré ayant de 4 à 20 atomes de carbone.

14. Composition selon l'une quelconque des revendications 1 à 10, caractérisée par le fait que ledit composé organique lipophile hydrofluorocarboné répond à la formule (V) :

$$C_rF_{2r+1}\text{-}(CH_2)_p\text{-}O\text{-}CO\text{-}(CH_2)_7\text{-}CH=CH\text{-}(CH_2)_7\text{-}CH_3 \qquad\qquad (V)$$

dans laquelle r est un nombre entier égal à 6 ou 8 et p est le nombre 1 ou 2.

15. Procédé de traitement cosmétique, caractérisé par le fait que l'on applique sur les cils ou sur les cheveux une composition telle que définie dans l'une quelconque des revendications précédentes.

16. Utilisation d'un composé organique lipophile hydrofluorocarboné tel que le rapport du nombre d'atomes de fluor au nombre total d'atomes de fluor et d'hydrogène liés aux atomes de carbone, dans la molécule dudit composé, est au moins égal à 10 % et n'est pas supérieur à 90 %, comme additif destiné à améliorer les propriétés d'une composition cosmétique comprenant une microdispersion aqueuse de cire.

17. Utilisation selon la revendication 16, dans laquelle ledit additif est destiné à améliorer la plasticité des films obtenus par application de ladite composition sur la peau.

18. Utilisation selon la revendication 16 ou 17, dans laquelle ladite composition est telle que définiè dans l'une quelconque des revendications 1 à 14.

**Claims**

1. Cosmetic composition or cosmetic composition carrier comprising an agueous microdispersion of particles containing at least one wax, the said particles being solid up to a temperature equal to at least 30°C, characterized in that the said particles contain, in addition, a lipophilic hydrofluorocarbon organic compound such that the proportion of the number of fluorine atoms with respect to the total number of fluorine and hydrogen atoms linked to the carbon atoms in the molecule of the said compound is equal to at least 10 % and is not greater than 90 %, the wax and the said lipophilic organic compound forming, in the said particles, a homogeneous mixture,
with the exception of a composition containing carnauba wax, an organofluorine compound, a nonionic surfactant and methyl para-hydroxybenzoate in the respective relative weight proportions p1/p2/p3/p4, the said nonionic surfactant being a glyceryl monostearate polyoxyethylenated with 30 mol of ethylene oxide and the said organofluorine compound being chosen from (a) 1-(2'-F-hexylethylthio)-3-(2''-ethylhexyloxy)propan-2-ol and (b) 1-(2'-F-octylethylthio)-2-hexanol,
on the understanding that:

EP 0 639 371 B1

in case (a) the said relative weight proportions p1/p2/p3/p4 are:
22.5/7.5/7.5/0.2 respectively
and that in case (b) the said relative weight proportions are 18/12/7.5/0.2 respectively.

2. Composition according to Claim 1, characterized in that the said proportion is within the range 10 to 80 %, and especially 30 to 60 %.

3. Composition according to either of the preceding claims, characterized in that it contains from 5 to 50 %, and especially from 10 to 30 %, of the said lipophilic hydrofluorocarbon organic compound, by weight relative to the total weight of wax present in the composition.

4. Composition according to any one of the preceding claims, characterized in that the said hydrofluorocarbon lipophilic organic compound is miscible with the carnauba wax to a level of at least 10 % by weight.

5. Composition according to any one of the preceding claims, characterized in that it contains from 1 to 40 % by weight, and especially from 5 to 30 % by weight, of wax.

6. Composition according to any one of the preceding claims, characterized in that the said lipophilic hydrofluorocarbon organic compound corresponds to the formula:

$$(R_F)_x(A)_y(R_H)_z \qquad\qquad (I)$$

in which:

$R_F$ represents a fluorinated organic group having at least four carbon atoms,
$R_H$ represents an unfluorinated hydrocarbon group, the said hydrocarbon group being optionally substituted and/or optionally containing one or more hetero atoms or heteroatomic groups,
A represents a divalent, trivalent or tetravalent linking group,
x represents the number 1, 2 or 3,
y represents the number 0 or 1,
z represents the number 0, 1, 2 or 3,
on the understanding:
that, when y is other than zero, the sum (x + z) is equal to the valency of the linking group A, and each group $R_F$ and $R_H$ is then linked to A via a covalent bond,
that, when y = 0, then x = z = 1 and the group $R_F$ and the group $R_H$ are then linked to one another via a covalent bond, and
that, when z is equal to 0, y is other than 0.

7. Composition according to the preceding claim, characterized in that $R_F$ is an optionally unsaturated aliphatic or an aromatic fluorocarbon group which can contain one or more hetero atoms or heteroatomic groups and/or which can be substituted with one or more halogen atoms other than fluorine, on the understanding that the proportion, in %, of the number of fluorine atoms with respect to the total number of halogen atoms present in $R_F$ is not less than 75 %.

8. Composition according to Claim 6, characterized in that $R_F$ represents a perfluoroalkyl group having from 4 to 22 carbon atoms.

9. Composition according to any one of Claims 6 to 8, characterized in that the linking group A is chosen from a carbon (tetravalent), a $\equiv$CH group (trivalent), a nitrogen (trivalent), a -CO-N= group (trivalent), an -SO$_2$N= group (trivalent), a PO-(O)$_3$- group (trivalent), or a divalent group chosen from -CH$_2$-, -O-, -S-, -SO-, -SO$_2$-and -CO-NH-.

10. Composition according to any one of Claims 6 to 9, characterized in that $R_H$ represents an optionally unsaturated 1-22 C alkyl group, a 6-10 C aryl group or a 7-15 C aralkyl group.

11. Composition according to any one of the preceding claims, characterized in that the said lipophilic hydrofluorocarbon organic compound corresponds to the formula (II):

$$R_1\text{-}(CH_2)_n\text{-}X\text{-}[C_3H_5(OH)]\text{-}(Y)_p\text{-}R_2 \qquad\qquad (II)$$

16

in which:

R$_1$ represents a perfluorinated alkyl radical having from 4 to 20 carbon atoms,

R$_2$ represents a C$_1$-C$_{22}$ alkyl, C$_6$-C$_{10}$ aryl or C$_7$-C$_{15}$ aralkyl group,

X and Y independently represent a group chosen from -O-, -S-, -SO-, -SO$_2$-,

n represents a number chosen from 0, 1, 2, 3 and 4,

p represents the number 0 or 1,

and C$_3$H$_5$(OH) represents one of the groups -CH$_2$CH(OH)CH$_2$-, -CH(CH$_2$OH)-CH$_2$- and -CH$_2$-CH(CH$_2$OH)-,

on the understanding that X and Y cannot simultaneously represent -SO- or -SO$_2$-.

12. Composition according to any one of Claims 1 to 9, characterized in that the said lipophilic hydrofluorocarbon organic compound corresponds to the formula (IV):

$$R_F\text{-}CH_2\text{-}CH_2\text{-}X\text{-}CH_2\text{-}CH(Y')\text{-}Z \qquad (IV)$$

in which:

R$_F$ is a perfluorinated alkyl group having from 4 to 20 carbon atoms,

X represents -O-, -S-, -SO- or -SO$_2$-,

either Y' represents OH, and Z then represents -C$_6$H$_5$, -CH$_3$, -CH$_2$OH, -CH$_2$O-COCH$_3$,

or Y' represents -CH$_2$OH, and Z then represents -O-COCH$_3$.

13. Composition according to any one of Claims 1 to 10, characterized in that the said lipophilic hydrofluorocarbon organic compound corresponds to the formula (III):

$$R_F\text{-}CH=CH\text{-}CH_2OCH_2\text{-}[C_2H_4\text{-}OW] \qquad (III)$$

in which:

C$_2$H$_4$-OW denotes -CHOH-CH$_2$W or -CHW-CH$_2$OH,

W represents -OR-, -SR-, -COOR, -OC$_6$H$_5$ or -O-C$_6$H$_4$-R",

R represents an alkyl group having 1 to 18 C,

R" represents -CH$_3$ or -OH at the ortho or para position, and

R$_F$ represents a perfluorinated alkyl radical having from 4 to 20 carbon atoms.

14. Composition according to any one of Claims 1 to 10, characterized in that the said lipophilic hydrofluorocarbon organic compound corresponds to the formula (V):

$$C_rF_{2r+1}\text{-}(CH_2)_p\text{-}O\text{-}CO\text{-}(CH_2)_7\text{-}CH=CH\text{-}(CH_2)_7\text{-}CH_3 \qquad (V)$$

in which r is an integer equal to 6 or 8 and p is the number 1 or 2.

15. Cosmetic treatment process, characterized in that a composition as defined in any one of the preceding claims is applied to eyelashes or to hair.

16. Use of a lipophilic hydrofluorocarbon organic compound such that the proportion of the number of fluorine atoms with respect to the total number of fluorine and hydrogen atoms linked to the carbon atoms in the molecule of the said compound is equal to at least 10 % and is not greater than 90 %, as an additive intended for improving the properties of a cosmetic composition comprising an aqueous microdispersion of wax.

17. Use according to Claim 16, in which the said additive is intended to improve the plasticity of films obtained by applying the said composition to the skin.

18. Use according to Claim 16 or 17, in which the said composition is as defined in any one of Claims 1 to 14.

**Patentansprüche**

1. Kosmetische Zusammensetzung oder Grundlage einer kosmetischen Zusammensetzung, enthaltend eine wäßrige Mikrodispersion von Teilchen, die mindestens ein Wachs enthalten, wobei diese Teilchen bis zu einer Temperatur von mindestens 30 °C fest sind, dadurch gekennzeichnet, daß die Teilchen zusätzlich eine lipophile, organische Fluorkohlenwasserstoffverbindung enthalten, bei der das Verhältnis der Anzahl an Fluoratomen oder der Gesamtzahl der Fluor- und Wasserstoffatome, die mit den Kohlenstoffatomen verbunden sind, in den Molekülen dieser Verbindung mindestens 10 % beträgt und 90 % nicht übersteigt, und das Wachs und die besagte lipophile, organische Verbindung in den Teilchen eine homogene Mischung bilden,

mit Ausnahme einer Zusammensetzung, die Carnaubawachs, eine fluororganische Verbindung, ein nichtionisches Tensid und Methyl-p-hydroxybenzoat in relativen Gewichtsanteilen von p1:p2:p3:p4 enthält, wobei das nichtionische Tensid ein Polyoxyethylenglycerolmonostearat mit 30 Mol Oxyethylen darstellt und die fluororganische Verbindung unter (a) 1-(2'-F-Hexylethylthio)-3-(2''-ethylhexyl-oxy)-propan-2-ol und (b) 1-(2'-F-Octylethylthio)-2-hexanol ausgewählt ist, und zwar für den Fall, daß (a) die relativen Gewichtsanteile p1:p2:p3:p4 = 22,5:7,5:7,5:0,2 betragen, und für den Fall, daß (b) die relativen Gewichtsanteile 18:12:7,5:0,2 betragen.

2. Zusammensetzung gemäß Anspruch 1, dadurch gekennzeichnet, daß das Verhältnis im Bereich von 10 bis 80 % und insbesondere von 30 bis 60 % liegt.

3. Zusammensetzung gemäß einem der vorstehenden Ansprüche, dadurch gekennzeichnet, daß sie 5 bis 50 % und insbesondere 10 bis 30 % der lipophilen, organischen Fluorkohlenstoffverbindung enthält, in Gewicht bezogen auf das Gesamtgewicht des in der Zusammensetzung vorhandenen Wachses.

4. Zusammensetzung gemäß einem der vorstehenden Ansprüche, dadurch gekennzeichnet, daß die lipophile, organische Fluorkohlenstoffverbindung mit Carnaubawachs in einer Menge von mindestens 10 Gew.-% mischbar ist.

5. Zusammensetzung gemäß einem der vorstehenden Ansprüche, dadurch gekennzeichnet, daß sie 1 bis 40 Gew.-% und insbesondere 5 bis 30 Gew.-% Wachs enthält.

6. Zusammensetzung gemäß einem der vorstehenden Ansprüche, dadurch gekennzeichnet, daß die lipophile, organische Fluorkohlenwasserstoffverbindung der Formel entspricht:

$$(R_F)_x(A)_y(R_H)_z \qquad\qquad (I)$$

in der bedeuten,

$R_F$ eine fluorierte organische Gruppe mit mindestens 4 Kohlenstoffatomen darstellt,

$R_H$ eine nichtfluorierte Kohlenwasserstoffgruppe bedeutet, wobei diese Kohlenwasserstoffgruppe gegebenenfalls substituiert ist und/oder gegebenenfalls eines oder mehrere Heteroatome oder Heteroatomgruppen trägt,

$A$ eine 2-, 3- oder 4-wertige Bindungsgruppe darstellt,

$x$ die Zahlen 1, 2 oder 3,

$y$ die Zahlen 0 oder 1, und

$z$ die Zahlen 0, 1, 2 oder 3

wobei

dann, wenn sich $y$ von Null unterscheidet, die Summe $(x + z)$ gleich der Valenz der Bindungsgruppe $A$ ist, und darüber hinaus die Gruppen $R_F$ und $R_H$ mit $A$ über eine kovalente Bindung verknüpft sind,

dann, wenn $y$ gleich Null ist, wiederum $x = z = 1$ gilt und die Gruppe $R_F$ und die Gruppe $R_H$ über eine kovalente Bindung miteinander verknüpft sind, und

dann, wenn $z = 0$ ist, sich $y$ von $0$ unterscheidet.

7. Zusammensetzung gemäß dem vorstehenden Anspruch, dadurch gekennzeichnet, daß $R_F$ eine gegebenenfalls ungesättigte, aliphatische oder aromatische Fluorkohlenwasserstoffgruppe ist, die ein oder mehrere Heteroatome oder Heteroatomgruppen tragen kann und/oder mit einem oder mehreren anderen Halogenatomen als Fluor substituiert sein kann, wobei die Menge (in Prozent) der Anzahl an Fluoratomen oder der Gesamtzahl an Halogenatomen, die in $R_F$ vorhanden sind, nicht unter 75 % liegt.

8. Zusammensetzung gemäß Anspruch 6, dadurch gekennzeichnet, daß $R_F$ eine Perfluoroalkylgruppe mit 4 bis 22 Kohlenstoffatomen darstellt.

9. Zusammensetzung gemäß einem der Ansprüche 6 bis 8, dadurch gekennzeichnet, däß die Bindungsgruppe A unter Kohlenstoff (4-wertig), einer Gruppe $\equiv$CH (dreiwertig), Stickstoff (dreiwertig), einer Gruppe -CO-N= (dreiwertig), einer Gruppe -SO$_2$N= (dreiwertig), einer Gruppe PO-(O)$_3$ (dreiwertig) oder einer unter -CH$_2$-, -O-, -S-, -SO-, -SO$_2$- und -CO-NH- ausgewählten, zweiwertigen Gruppe ausgewählt ist.

10. Zusammensetzung gemäß einem der Ansprüche 6 bis 9, dadurch gekennzeichnet, daß $R_H$ eine gegebenenfalls ungesättigte $C_{1-22}$-Alkylgruppe, $C_{6-10}$-Arylgruppe oder $C_{7-15}$- Aralkylgruppe darstellt.

11. Zusammensetzung gemäß einem der vorstehenden Ansprüche, dadurch gekennzeichnet, daß die lipophile, organische Fluorkohlenwasserstoffverbindung der Formel (II) entspricht:

$$R_1\text{-}(CH_2)_n\text{-}X\text{-}[C_3H_5(OH)]\text{-}(Y)_p\text{-}R_2 \tag{II}$$

in der

| | |
|---|---|
| $R_1$ | einen perfluorierten Alkylrest mit 4 bis 20 Kohlenstoffatomen, |
| $R_2$ | eine $C_1$-$C_{22}$-Alkylgruppe, eine $C_6$-$C_{10}$-Arylgruppe oder eine $C_7$-$C_{15}$-Aralkylgruppe darstellt, |
| X und Y | unabhängig voneinander eine Gruppe ausgewählt unter -O-, -S-, -SO- und -SO$_2$- bedeuten, |
| n | eine unter 0, 1, 2, 3 oder 4 ausgewählte Zahl bedeutet, |
| p | die Zähl 0 oder 1 bedeuten, und |
| $C_3H_5(OH)$ | für die Gruppen -CH$_2$CH(OH)CH$_2$-, -CH(CH$_2$OH-CH$_2$- und -CH$_2$-CH(CH$_2$OH)- steht, wobei X und Y nicht gleichzeitig -SO- oder -SO$_2$- sein können. |

12. Zusammensetzung gemäß einem der Ansprüche 1 bis 9, dadurch gekennzeichnet, daß die lipophile, organische Fluorkohlenwasserstoffverbindung der Formel (IV) entspricht:

$$R_F\text{-}CH_2\text{-}CH_2\text{-}X\text{-}CH_2\text{-}CH(Y')\text{-}Z \tag{IV}$$

in der

| | |
|---|---|
| $R_F$ | eine perfluorierte Akylgruppe mit 4 bis 20 Kohlenstoffatomen darstellt, |
| X | -O-, -S-, -SO- oder -SO$_2$ darstellt, und |
| Z dann, | wenn Y' für OH steht, -C$_6$H$_5$-, -CH$_3$, -CH$_2$OH, -CH$_2$O-COCH$_3$ be-deutet, und |
| Z dann, | wenn Y' für -CH$_2$OH steht, -O-COCH$_3$ bedeutet. |

13. Zusammensetzung gemäß einem der Ansprüche 1 bis 10, dadurch gekennzeichnet, daß die lipophile, organische Fluorkohlenwasserstoffverbindung der Formel (III) entspricht:

$$R_F\text{-}CH=CH\text{-}CH_2OCH2\text{-}[C_2H_4\text{-}OW] \tag{III}$$

in der

| | |
|---|---|
| $C_2H_4$-OW | -CHOH-CH$_2$W oder -CHW-CH$_2$OH bedeutet, |
| W | -OR, -SR, -COOR, -OC$_6$H$_5$ oder -O-C$_6$H$_4$-R'' darstellt, |
| R | für eine Alkylgruppe mit 1 bis 18 Kohlenstoffatomen steht, |
| R'' | -CH$_3$ oder -OH in ortho- oder para-Position darstellt, und |
| $R_F$ | einen perfluorierten Alkylrest mit 4 bis 20 Kohlenstoffatomen darstellt. |

14. Zusammensetzung gemäß einem der Ansprüche 1 bis 10, dadurch gekennzeichnet, daß die lipophile, organische Fluorkohlenwasserstoffverbindung der Formel (V) entspricht:

$$C_rF_{2r+1}\text{-}(CH_2)_p\text{-}O\text{-}CO\text{-}(CH_2)_7\text{-}CH=CH\text{-}(CH_2)_7\text{-}CH_3 \tag{V}$$

in der

r   eine ganze Zähl gleich 6 oder 8, und

p   die Zahlen 1 oder 2 bedeutet.

**15.** Kosmetisches Behandlungsverfahren, dadurch gekennzeichnet, daß man auf Wimpern oder Haare eine Zusammensetzung aufträgt, wie sie in einem der vorstehenden Ansprüche definiert ist.

**16.** Verwendung einer lipophilen, organischen Hydrofluorkohlenwasserstoffverbindung, in der das Verhältnis der Anzahl der Fluoratome oder der Gesamtzähl der Fluoratome und Wasserstoffe, die mit den Kohlenstoffatomen verbunden sind, in dem Molekül der Verbindung mindestens 10 % beträgt und 90 % nicht übersteigt, als Additive zur Verbesserung der Eigenschaften einer kosmetischen Zusammensetzung, die eine wäßrige Wachs-Mikrodispersion enthält.

**17.** Verwendung gemäß Anspruch 16, in der das Additiv zur Verbesserung der Plastizität von Filmen dient, die durch Auftragen der Zusammensetzung auf die Haut erhalten werden.

**18.** Verwendung gemäß den Ansprüchen 16 oder 17, bei der die Zusammensetzung so, wie in einem der Ansprüche 1 bis 14 definiert, beschaffen ist.